# EUROPEAN PATENT APPLICATION

(11) **EP 4 759 235 A1**
(43) Date of publication of application: **17.06.2026**
(21) Application number: 25218775.2
(22) Date of filing: 26.11.2025
(51) Int. Cl.: A61B 5/397, A61B 5/00, G06F 3/01

(54) **BAND-TO-CAPSULE CONNECTION TECHNIQUES AND ASSEMBLY METHODS FOR A WRIST-WEARABLE DEVICE**

(30) Priority: 10.12.2024 US 202463730386 P; 21.11.2025 US 202519397635
(71) Applicant: Meta Platforms Technologies, LLC, Menlo Park, CA 94025 (US)
(72) Inventor: PAISNER, Rachel, Menlo Park, 94025 (US); MOREIRA, Claudia, Menlo Park, 94025 (US); AGGARWAL, Shubham, Menlo Park, 94025 (US); GARCIA, Michael, Menlo Park, 94025 (US); ROSENKRANZ, Andrew, Menlo Park, 94025 (US); ZHAO, Wancheng, Menlo Park, 94025 (US); APARICIO, Edwin Corona, Menlo Park, 94025 (US); CAPUL, Marc, Menlo Park, 94025 (US); DOCHERTY, Sean, Menlo Park, 94025 (US); AUCLAIR, Martin, Menlo Park, 94025 (US); KOWALSKI, Stefan Ahldor, Menlo Park, 94025 (US); ISLAM, Md Rashidul, Menlo Park, 94025 (US); LIU, Chia-Wei, Menlo Park, 94025 (US); SHIN, Joung Sub, Menlo Park, 94025 (US); ARDISANA, John Bernard, Menlo Park, 94025 (US)
(74) Representative: Murgitroyd & Company

(57) **Abstract**

An example band portion of a wrist-wearable device includes a flexible printed circuit (FPC) board, a mounting plate, a structural component coupled to a capsule portion of the wrist-wearable device, and one or more electrodes. The one or more electromyography sensors each include one or more electrodes configured to couple to the FPC board. The wrist-wearable device also includes a strain relief layer coupled to the FPC board. A first portion of the strain relief layer is configured to secure the mounting plate within the strain relief layer and the mounting plate includes at least one recess for engaging the mounting plate with a structural component of the band portion, the mounting plate configured to couple the strain relief layer to the structural component.

## Description

### TECHNICAL FIELD

This relates generally to the placement and assembly of a wrist-wearable device that is used as an input device for interacting with an extended-reality environment.

### BACKGROUND

Wrist-wearable devices that include sensors in the band are large on the user's wrist and are difficult to manufacture at scale. Moreover, these wrist-wearable devices struggle to last long term as the sensitive sensors cannot handle the repeated donning and doffing of the wrist-wearable device.

As such, there is a need to address one or more of the above-identified challenges. A brief summary of solutions to the issues noted above are described below.

### SUMMARY

According to an aspect, there is provided a band portion of a wrist-wearable device, comprising:
a flexible printed circuit, FPC, board;
a mounting plate;
a structural component coupled to a capsule portion of the wrist-wearable device;
one or more electromyography sensors, wherein the one or more electromyography sensors each include one or more electrodes configured to couple to the FPC board; and
a strain relief layer coupled to the FPC board, wherein:
   a first portion of the strain relief layer wraps around at least the mounting plate such that the first portion of the strain relief layer is folded back onto and coupled to a second portion of the strain relief layer to secure the mounting plate within the strain relief layer; and
   the mounting plate includes at least one recess for engaging the mounting plate with a structural component of the band portion, the mounting plate configured to couple the strain relief layer to the structural component.

In one embodiment, the strain relief layer comprises a multifilament yarn spun from liquid crystal polymer, LCP.

In one embodiment, the first portion of the strain relief layer is coupled to the second portion of the strain relief layer via an adhesive.

In one embodiment, the adhesive is a heat-activated adhesive, HAF.

In one embodiment, the mounting plate is engaged with the structural component when the recess of the mounting plate is partially surrounding a pin of the structural component, where the pin secured via laser welding.

In one embodiment, a stiffening material is placed between the FPC board and the strain relief layer.

In one embodiment, the stiffening material is coupled via an adhesive to the FPC and the strain relief layer.

In one embodiment, the strain relief layer incudes one or more cutouts for placing the FPC board.

In one embodiment, the band portion is overmolded by liquid silicone rubber that encapsulates some of the band portion of the wrist-wearable device.

In one embodiment, overmolding is a two shot overmolding process.

In one embodiment, the band portion includes a sealing gasket for inhibiting moisture and debris ingress at an electrical connection point between the band portion and a capsule portion of the wrist-wearable device.

In one embodiment, the strain relief layer incudes one or more cutouts for placing a flexible printed circuit board.

According to another aspect, there is provided a wrist-wearable device comprising:
a band portion including one or more electrical components, wherein the band portion is configured to couple to a capsule portion with one or more additional electronic components;
a multifilament yarn spun from liquid crystal polymer, LCP, layer coupled to the band portion and coupled to the one or more electrical components of the band portion;
a mounting plate coupled to a first end of the multifilament yarn spun from LCP layer, wherein:
   a first end of the LCP layer is configured to wrap around the mounting plate back onto the LCP layer such that the first end of the LCP layer is configured to couple to a portion of the LCP layer via an adhesive; and
   a connector piece configured to couple to the mounting plate via pins.

In one embodiment, the adhesive is a heat-activated adhesive, HAF.

In one embodiment, the band portion is overmolded by liquid silicone rubber that encapsulates some of the band portion of the wrist-wearable device.

In one embodiment, overmolding is a two shot overmolding process.

In one embodiment, the band portion includes a sealing gasket for inhibiting moisture and debris ingress at an electrical connection point between the band portion and a capsule portion of the wrist-wearable device.

According to a further aspect, there is provided a method, comprising:
coupling a strain relief layer to a mounting plate via wrapping a first portion of the strain relief layer around the mounting plate such that the first portion of the strain relief layer is folded back onto and coupled to a second portion of the strain relief layer; and
engaging the mounting plate with a structural component of a band portion of a wrist-wearable device via a recess of the mounting plate and a pin of the structural component.

In one embodiment, the method further comprises coupling the structural component of the band portion to a capsule portion of a wrist-wearable device.

In one embodiment, engaging the mounting plate with the structural component of the band portion includes coupling the recess of the mounting plate such that the recess partially surrounds the pin of the structural component and the method further includes coupling another recess of the mounting plate such that the other recess partially surrounds another pin of the structural component.

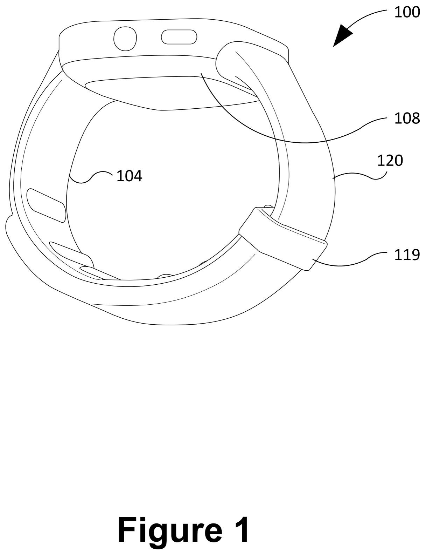

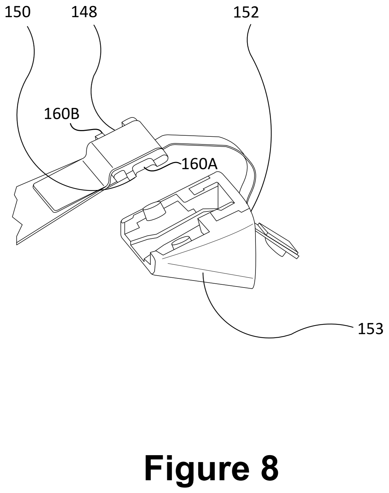

An example band for a wrist-wearable device is described herein, in which the band can withstand repeated use and has form factor that is not cumbersome when worn on a wrist of a user. An example band portion of a wrist-wearable device comprises a flexible printed circuit (FPC) board (e.g., Figure 1 illustrates a flexible printed circuit 128). The band portion includes a mounting plate, a structural component coupled to a capsule portion of the wrist-wearable device, and one or more electrodes of electromyography sensors configured to couple to the flexible printed circuit board (e.g., Figure 1 illustrates a plurality of electrode receivers 114A-114F that are configured to receive a plurality of electrodes that are configured to record neuromuscular signals of a wearer). The band portion also includes a strain relief layer that is coupled to the flexible printed circuit board (e.g., Figure 1 shows a strain relief layer 126 comprised of a multifilament yarn spun from a liquid crystal polymer sheet). In some embodiments, a first portion of the strain relief layer wraps around the mounting plate such that the first portion of the strain relief layer is folded back onto and coupled to a second portion of the strain relief layer to secure the mounting plate within the strain relief layer and the mounting plate includes at least one recess for engaging the mounting plate with a structural component of the band portion, the mounting plate configured to couple the strain relief layer to the structural component. For example, Figures 8-9 show that the strain relief layer encapsulates a mounting plate 150 that interfaces structural component 152 that interfaces with the capsule portion 108.

The devices and/or systems described herein can be configured to include instructions that cause the performance of methods and operations associated with the presentation and/or interaction with an extended-reality (XR) headset. These methods and operations can be stored on a non-transitory computer-readable storage medium of a device or a system. It is also noted that the devices and systems described herein can be part of a larger, overarching system that includes multiple devices. A non-exhaustive of list of electronic devices that can, either alone or in combination (e.g., a system), include instructions that cause the performance of methods and operations associated with the presentation and/or interaction with an XR experience include an extended-reality headset (e.g., a mixed-reality (MR) headset or a pair of augmented-reality (AR) glasses as two examples), a wrist-wearable device, an intermediary processing device, a smart textile-based garment, etc. For example, when an XR headset is described, it is understood that the XR headset can be in communication with one or more other devices (e.g., a wrist-wearable device, a server, intermediary processing device) which together can include instructions for performing methods and operations associated with the presentation and/or interaction with an extended-reality system (i.e., the XR headset would be part of a system that includes one or more additional devices). Multiple combinations with different related devices are envisioned, but not recited for brevity.

In addition, the example band described herein also is configured to withstand everyday environmental factors, such as moisture and debris ingress resistance (e.g., through the use of an O-ring gasket used between a connection point between the band portion and capsule portion of the wrist-wearable device).

It will be appreciated that any features described herein as being suitable for incorporation into one or more aspects or embodiments of the present disclosure are intended to be generalizable across any and all aspects and embodiments of the present disclosure. Other aspects of the present disclosure can be understood by those skilled in the art in light of the description, the claims, and the drawings of the present disclosure. The foregoing general description and the following detailed description are exemplary and explanatory only and are not restrictive of the claims.

### BRIEF DESCRIPTION OF THE DRAWINGS

For a better understanding of the various described embodiments, reference should be made to the Detailed Description below, in conjunction with the following drawings in which like reference numerals refer to corresponding parts throughout the figures.
Figures 1-3 illustrate a wrist-wearable device that includes a band portion that includes one or more electrodes, in accordance with some embodiments.
Figures 2-4 illustrate the middle layer that was described in reference to Figures 1-3 in further detail, in accordance with some embodiments.
Figures 5-8 illustrate how a strain relief layer is attached to a structural component that interfaces with the capsule portion of the wrist-wearable device, in accordance with some embodiments.
Figures 9-11 show the interfacing portion of the band portion of the wrist-wearable device, in accordance with some embodiments.
Figures 12A, 12B, 12C-1 and 12C-2 illustrate example MR and AR systems, in accordance with some embodiments.

In accordance with common practice, the various features illustrated in the drawings may not be drawn to scale. Accordingly, the dimensions of the various features may be arbitrarily expanded or reduced for clarity. In addition, some of the drawings may not depict all of the components of a given system, method, or device. Finally, like reference numerals may be used to denote like features throughout the specification and figures.

### DETAILED DESCRIPTION

Numerous details are described herein to provide a thorough understanding of the example embodiments illustrated in the accompanying drawings. However, some embodiments may be practiced without many of the specific details, and the scope of the claims is only limited by those features and aspects specifically recited in the claims. Furthermore, well-known processes, components, and materials have not necessarily been described in exhaustive detail so as to avoid obscuring pertinent aspects of the embodiments described herein.

### Overview

Embodiments of this disclosure can include or be implemented in conjunction with various types of extended-realities (XRs) such as mixed-reality (MR) and augmented-reality (AR) systems. MRs and ARs, as described herein, are any superimposed functionality and/or sensory-detectable presentation provided by MR and AR systems within a user's physical surroundings. Such MRs can include and/or represent virtual realities (VRs) and VRs in which at least some aspects of the surrounding environment are reconstructed within the virtual environment (e.g., displaying virtual reconstructions of physical objects in a physical environment to avoid the user colliding with the physical objects in a surrounding physical environment). In the case of MRs, the surrounding environment that is presented through a display is captured via one or more sensors configured to capture the surrounding environment (e.g., a camera sensor, time-of-flight (ToF) sensor). While a wearer of an MR headset can see the surrounding environment in full detail, they are seeing a reconstruction of the environment reproduced using data from the one or more sensors (i.e., the physical objects are not directly viewed by the user). An MR headset can also forgo displaying reconstructions of objects in the physical environment, thereby providing a user with an entirely VR experience. An AR system, on the other hand, provides an experience in which information is provided, e.g., through the use of a waveguide, in conjunction with the direct viewing of at least some of the surrounding environment through a transparent or semi-transparent waveguide(s) and/or lens(es) of the AR glasses. Throughout this application, the term "extended reality (XR)" is used as a catchall term to cover both ARs and MRs. In addition, this application also uses, at times, a head-wearable device or headset device as a catchall term that covers XR headsets such as AR glasses and MR headsets.

As alluded to above, an MR environment, as described herein, can include, but is not limited to, non-immersive, semi-immersive, and fully immersive VR environments. As also alluded to above, AR environments can include marker-based AR environments, markerless AR environments, location-based AR environments, and projection-based AR environments. The above descriptions are not exhaustive and any other environment that allows for intentional environmental lighting to pass through to the user would fall within the scope of an AR, and any other environment that does not allow for intentional environmental lighting to pass through to the user would fall within the scope of an MR.

The AR and MR content can include video, audio, haptic events, sensory events, or some combination thereof, any of which can be presented in a single channel or in multiple channels (such as stereo video that produces a three-dimensional effect to a viewer). Additionally, AR and MR can also be associated with applications, products, accessories, services, or some combination thereof, which are used, for example, to create content in an AR or MR environment and/or are otherwise used in (e.g., to perform activities in) AR and MR environments.

Interacting with these AR and MR environments described herein can occur using multiple different modalities and the resulting outputs can also occur across multiple different modalities. In one example AR or MR system, a user can perform a swiping in-air hand gesture to cause a song to be skipped by a song-providing application programming interface (API) providing playback at, for example, a home speaker.

A hand gesture, as described herein, can include an in-air gesture, a surface-contact gesture, and or other gestures that can be detected and determined based on movements of a single hand (e.g., a one-handed gesture performed with a user's hand that is detected by one or more sensors of a wearable device (e.g., electromyography (EMG) and/or inertial measurement units (IMUs) of a wrist-wearable device, and/or one or more sensors included in a smart textile wearable device) and/or detected via image data captured by an imaging device of a wearable device (e.g., a camera of a head-wearable device, an external tracking camera setup in the surrounding environment)). "In-air" generally includes gestures in which the user's hand does not contact a surface, object, or portion of an electronic device (e.g., a head-wearable device or other communicatively coupled device, such as the wrist-wearable device), in other words the gesture is performed in open air in 3D space and without contacting a surface, an object, or an electronic device. Surface-contact gestures (contacts at a surface, object, body part of the user, or electronic device) more generally are also contemplated in which a contact (or an intention to contact) is detected at a surface (e.g., a single- or double-finger tap on a table, on a user's hand or another finger, on the user's leg, a couch, a steering wheel). The different hand gestures disclosed herein can be detected using image data and/or sensor data (e.g., neuromuscular signals sensed by one or more biopotential sensors (e.g., EMG sensors) or other types of data from other sensors, such as proximity sensors, ToF sensors, sensors of an IMU, capacitive sensors, strain sensors) detected by a wearable device worn by the user and/or other electronic devices in the user's possession (e.g., smartphones, laptops, imaging devices, intermediary devices, and/or other devices described herein).

The input modalities as alluded to above can be varied and are dependent on a user's experience. For example, in an interaction in which a wrist-wearable device is used, a user can provide inputs using in-air or surface-contact gestures that are detected using neuromuscular signal sensors of the wrist-wearable device. In the event that a wrist-wearable device is not used, alternative and entirely interchangeable input modalities can be used instead, such as camera(s) located on the headset/glasses or elsewhere to detect in-air or surface-contact gestures or inputs at an intermediary processing device (e.g., through physical input components (e.g., buttons and trackpads)). These different input modalities can be interchanged based on both desired user experiences, portability, and/or a feature set of the product (e.g., a low-cost product may not include hand-tracking cameras).

While the inputs are varied, the resulting outputs stemming from the inputs are also varied. For example, an in-air gesture input detected by a camera of a head-wearable device can cause an output to occur at a head-wearable device or control another electronic device different from the head-wearable device. In another example, an input detected using data from a neuromuscular signal sensor can also cause an output to occur at a head-wearable device or control another electronic device different from the head-wearable device. While only a couple examples are described above, one skilled in the art would understand that different input modalities are interchangeable along with different output modalities in response to the inputs.

Specific operations described above may occur as a result of specific hardware. The devices described are not limiting and features on these devices can be removed or additional features can be added to these devices. The different devices can include one or more analogous hardware components. For brevity, analogous devices and components are described herein. Any differences in the devices and components are described below in their respective sections.

As described herein, a processor (e.g., a central processing unit (CPU) or microcontroller unit (MCU)), is an electronic component that is responsible for executing instructions and controlling the operation of an electronic device (e.g., a wrist-wearable device, a head-wearable device, a handheld intermediary processing device (HIPD), a smart textile-based garment, or other computer system). There are various types of processors that may be used interchangeably or specifically required by embodiments described herein. For example, a processor may be (i) a general processor designed to perform a wide range of tasks, such as running software applications, managing operating systems, and performing arithmetic and logical operations; (ii) a microcontroller designed for specific tasks such as controlling electronic devices, sensors, and motors; (iii) a graphics processing unit (GPU) designed to accelerate the creation and rendering of images, videos, and animations (e.g., VR animations, such as three-dimensional modeling); (iv) a field-programmable gate array (FPGA) that can be programmed and reconfigured after manufacturing and/or customized to perform specific tasks, such as signal processing, cryptography, and machine learning; or (v) a digital signal processor (DSP) designed to perform mathematical operations on signals such as audio, video, and radio waves. One of skill in the art will understand that one or more processors of one or more electronic devices may be used in various embodiments described herein.

As described herein, controllers are electronic components that manage and coordinate the operation of other components within an electronic device (e.g., controlling inputs, processing data, and/or generating outputs). Examples of controllers can include (i) microcontrollers, including small, low-power controllers that are commonly used in embedded systems and Internet of Things (IoT) devices; (ii) programmable logic controllers (PLCs) that may be configured to be used in industrial automation systems to control and monitor manufacturing processes; (iii) system-on-a-chip (SoC) controllers that integrate multiple components such as processors, memory, I/O interfaces, and other peripherals into a single chip; and/or (iv) DSPs. As described herein, a graphics module is a component or software module that is designed to handle graphical operations and/or processes and can include a hardware module and/or a software module.

As described herein, memory refers to electronic components in a computer or electronic device that store data and instructions for the processor to access and manipulate. The devices described herein can include volatile and non-volatile memory. Examples of memory can include (i) random access memory (RAM), such as DRAM, SRAM, DDR RAM or other random access solid state memory devices, configured to store data and instructions temporarily; (ii) read-only memory (ROM) configured to store data and instructions permanently (e.g., one or more portions of system firmware and/or boot loaders); (iii) flash memory, magnetic disk storage devices, optical disk storage devices, other non-volatile solid state storage devices, which can be configured to store data in electronic devices (e.g., universal serial bus (USB) drives, memory cards, and/or solid-state drives (SSDs)); and (iv) cache memory configured to temporarily store frequently accessed data and instructions. Memory, as described herein, can include structured data (e.g., SQL databases, MongoDB databases, GraphQL data, or JSON data). Other examples of memory can include (i) profile data, including user account data, user settings, and/or other user data stored by the user; (ii) sensor data detected and/or otherwise obtained by one or more sensors; (iii) media content data including stored image data, audio data, documents, and the like; (iv) application data, which can include data collected and/or otherwise obtained and stored during use of an application; and/or (v) any other types of data described herein.

As described herein, a power system of an electronic device is configured to convert incoming electrical power into a form that can be used to operate the device. A power system can include various components, including (i) a power source, which can be an alternating current (AC) adapter or a direct current (DC) adapter power supply; (ii) a charger input that can be configured to use a wired and/or wireless connection (which may be part of a peripheral interface, such as a USB, micro-USB interface, near-field magnetic coupling, magnetic inductive and magnetic resonance charging, and/or radio frequency (RF) charging); (iii) a power-management integrated circuit, configured to distribute power to various components of the device and ensure that the device operates within safe limits (e.g., regulating voltage, controlling current flow, and/or managing heat dissipation); and/or (iv) a battery configured to store power to provide usable power to components of one or more electronic devices.

As described herein, peripheral interfaces are electronic components (e.g., of electronic devices) that allow electronic devices to communicate with other devices or peripherals and can provide a means for input and output of data and signals. Examples of peripheral interfaces can include (i) USB and/or micro-USB interfaces configured for connecting devices to an electronic device; (ii) Bluetooth interfaces configured to allow devices to communicate with each other, including Bluetooth low energy (BLE); (iii) near-field communication (NFC) interfaces configured to be short-range wireless interfaces for operations such as access control; (iv) pogo pins, which may be small, spring-loaded pins configured to provide a charging interface; (v) wireless charging interfaces; (vi) global-positioning system (GPS) interfaces; (vii) Wi-Fi interfaces for providing a connection between a device and a wireless network; and (viii) sensor interfaces.

As described herein, sensors are electronic components (e.g., in and/or otherwise in electronic communication with electronic devices, such as wearable devices) configured to detect physical and environmental changes and generate electrical signals. Examples of sensors can include (i) imaging sensors for collecting imaging data (e.g., including one or more cameras disposed on a respective electronic device, such as a simultaneous localization and mapping (SLAM) camera); (ii) biopotential-signal sensors; (iii) IMUs for detecting, for example, angular rate, force, magnetic field, and/or changes in acceleration; (iv) heart rate sensors for measuring a user's heart rate; (v) peripheral oxygen saturation (SpO2) sensors for measuring blood oxygen saturation and/or other biometric data of a user; (vi) capacitive sensors for detecting changes in potential at a portion of a user's body (e.g., a sensor-skin interface) and/or the proximity of other devices or objects; (vii) sensors for detecting some inputs (e.g., capacitive and force sensors); and (viii) light sensors (e.g., ToF sensors, infrared light sensors, or visible light sensors), and/or sensors for sensing data from the user or the user's environment. As described herein biopotential-signal-sensing components are devices used to measure electrical activity within the body (e.g., biopotential-signal sensors). Some types of biopotential-signal sensors include (i) electroencephalography (EEG) sensors configured to measure electrical activity in the brain to diagnose neurological disorders; (ii) electrocardiography (ECG or EKG) sensors configured to measure electrical activity of the heart to diagnose heart problems; (iii) EMG sensors configured to measure the electrical activity of muscles and diagnose neuromuscular disorders; (iv) electrooculography (EOG) sensors configured to measure the electrical activity of eye muscles to detect eye movement and diagnose eye disorders.

As described herein, an application stored in memory of an electronic device (e.g., software) includes instructions stored in the memory. Examples of such applications include (i) games; (ii) word processors; (iii) messaging applications; (iv) media-streaming applications; (v) financial applications; (vi) calendars; (vii) clocks; (viii) web browsers; (ix) social media applications; (x) camera applications; (xi) web-based applications; (xii) health applications; (xiii) AR and MR applications; and/or (xiv) any other applications that can be stored in memory. The applications can operate in conjunction with data and/or one or more components of a device or communicatively coupled devices to perform one or more operations and/or functions.

As described herein, communication interface modules can include hardware and/or software capable of data communications using any of a variety of custom or standard wireless protocols (e.g., IEEE 802.15.4, Wi-Fi, ZigBee, 6LoWPAN, Thread, Z-Wave, Bluetooth Smart, ISA100.11a, WirelessHART, or MiWi), custom or standard wired protocols (e.g., Ethernet or HomePlug), and/or any other suitable communication protocol, including communication protocols not yet developed as of the filing date of this document. A communication interface is a mechanism that enables different systems or devices to exchange information and data with each other, including hardware, software, or a combination of both hardware and software. For example, a communication interface can refer to a physical connector and/or port on a device that enables communication with other devices (e.g., USB, Ethernet, HDMI, or Bluetooth). A communication interface can refer to a software layer that enables different software programs to communicate with each other (e.g., APIs and protocols such as HTTP and TCP/IP).

As described herein, a graphics module is a component or software module that is designed to handle graphical operations and/or processes and can include a hardware module and/or a software module.

As described herein, non-transitory computer-readable storage media are physical devices or storage medium that can be used to store electronic data in a non-transitory form (e.g., such that the data is stored permanently until it is intentionally deleted and/or modified).

### Band Portion of Wrist Wearable Device

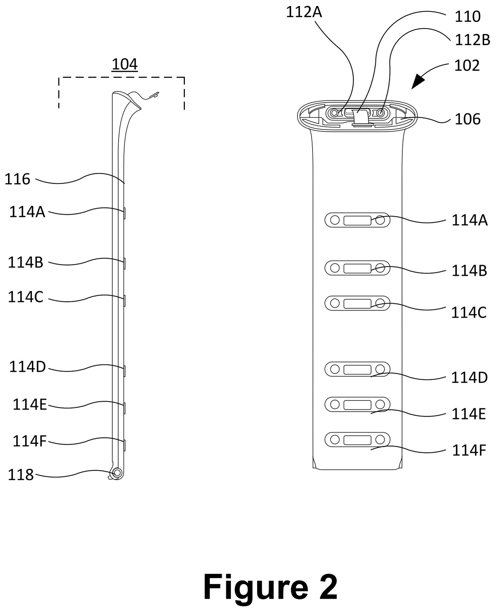

Figure 1 illustrates a wrist-wearable device that includes a band portion that includes one or more electrodes, in accordance with some embodiments. Figure 1 shows an example wrist-wearable device 100 that includes multiple bands, where at least one of the bands includes one or more electrodes configured to record electromyography data of a wearer. Figure 2 shows a top-down view 102 and a profile view 104 of a band portion 120. The top-down view 102 of the band portion shows an interface region 106 in which the band portion can be coupled to a capsule portion 108, as shown in the example wrist-wearable device 100 in Figure 1. The interface region also includes a data connection cable 110 and fastening locations 112A and 112B (e.g., threaded screw holes, rivet locations, etc.). The top-down view 102 also shows a region in which electrodes (not pictured) can be coupled to a plurality of electrode receivers 114A-114F. As shown in profile view 104, the electrode receivers are substantially flush with the surface 116 of the band portion 120. In some embodiments, the band portion can also include a region 118 at the distal end for receiving a loop 119 for guiding the other band portion 120 around the wrist of a user.

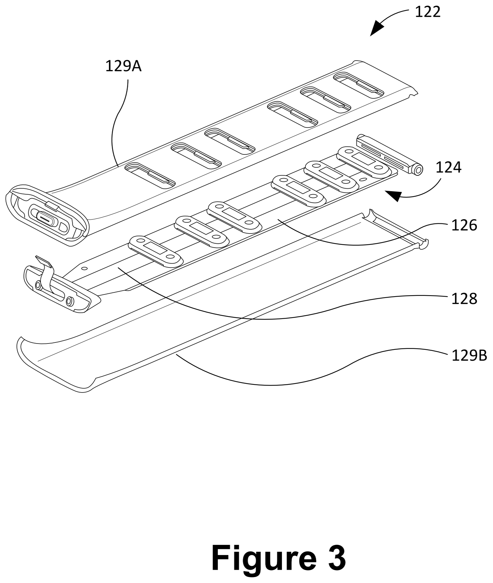

Figure 3 also shows a partial exploded view 122 that illustrates the construction of the band portion 120. In the partial exploded view, a middle layer 124 is shown which includes a strain relief layer 126 that is comprised of a multifilament yarn spun from a liquid crystal polymer sheet (e.g., Vectran), and also includes a flexible printed circuit 128 that is electrically coupled to the plurality of electrode receivers 114A-114F. The middle layer 124 is encased by an injection-molded liquid silicone rubber (LSR), which is constructed using two separate shots of the LSR material (top shot 129A and bottom shot 129B). In some embodiments, the LSR materials are the same hardness, e.g., having a shore hardness of 50-80A. In some embodiments, the LSR materials differ and the hardness varies based on the respective material and whether the material undergoes more deformation when donned.

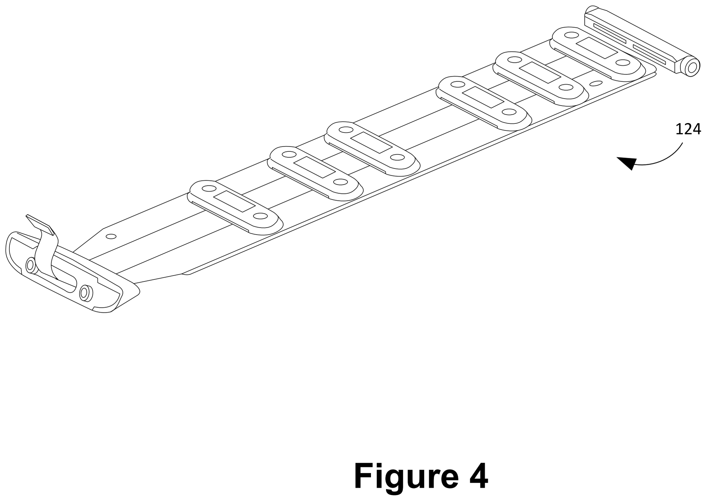

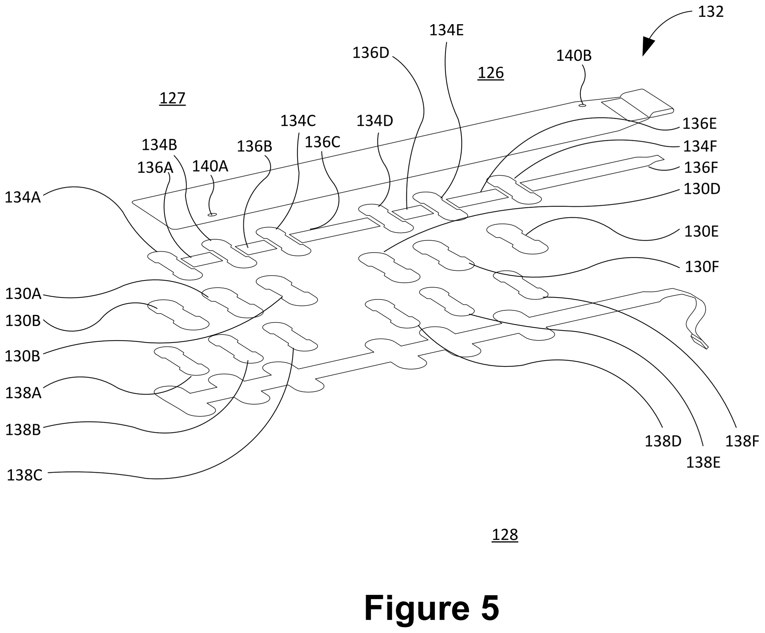

Figure 4 illustrates the middle layer 124 that was described in reference to Figure 3 in further detail, in accordance with some embodiments. Figure 5 shows an exploded view 132 of the middle layer 124, which includes a plurality of subcomponents, in accordance with some embodiments. The subcomponents of the middle layer include a strain relief layer 126 comprised of a multifilament yarn spun from a liquid crystal polymer sheet. The strain relief layer 126 has pressure-sensitive adhesive (PSA) 127 placed on it for coupling the strain relief layer 126 to both stiffening material 130A-130F and the flexible printed circuit 128. In some embodiments, the PSA is not a continuous piece and has varying sizes based on what the PSA is bonding to. For example, PSA components 134A-134F that bond with the stiffening material 130A-130F have a first shape and thickness (e.g., a shape matching the shape of an electrode with a first thickness (e.g., 0.05 mm)) and PSA components 136A-136F that directly bond with the flexible printed circuit 128 have different shapes and thickness than the PSA components 134A-134F (e.g., PSA components 136A-136F have a second thickness of 0.125 mm). The exact count of each PSA component is variable and based on the number of electrodes used required for the electromyography sensor. Figure 5 also shows another set of PSA components 138A-138F that are placed on the other side of the stiffening material 130A-130F and bond to the flexible printed circuit 128. In some embodiments, the PSA components are also stiffening components and contribute to the overall structural rigidity needed to ensure excessive deformation is not caused to the flexible printed circuit 128. In some embodiments, the combined thickness of the PSA components 134A-134F, stiffening material 130A-130F, and the other set of PSA components 138A-138F, is equal to the thickness of the PSA components 135A-136F. Having a uniform thickness provides a flat surface for the flexible circuit to couple with while ensuring areas that need to have more or less rigidity are able to do so. In some embodiments, the strain relief layer 126 includes one or more cutouts 140A-140B that guide one or more of the PSA components, stiffening components, and/or the flexible circuit. In some embodiments, all the PSAs described can be interchanged with heat-activated films (HAFs). In some embodiments, a combination of PSAs and HAFs are used based on the different manufacturing requirements.

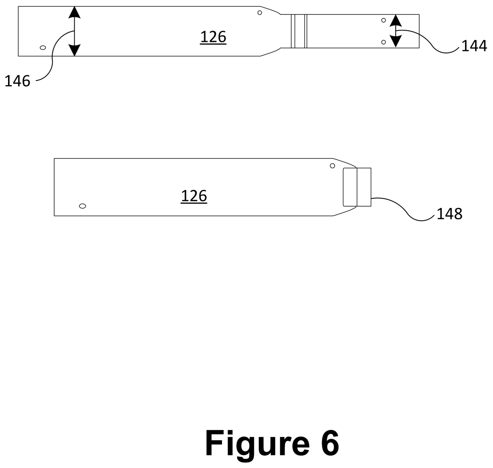

Figure 6 illustrates how strain relief layer 126 is attached to a structural component 152 that interfaces with the capsule portion 108 (shown in Figure 1) of the wrist-wearable device 100 (shown in Figure 1), in accordance with some embodiments. As shown in Figure 6, the strain relief layer 126 includes a first section width 144 and a second section width 146. The second illustration shows that the strain relief layer can be folded over itself to produce a loop 148, and in some embodiments excess material can be trimmed off. The loop 148 has a section width that corresponds with the first section width 144 and the first section width 144 is less than the second section width 146.

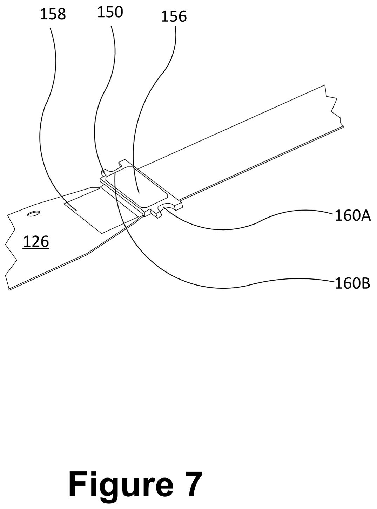

Figures 7-9 also shows a sequence in which the loop 148 encases a mounting plate 150 for coupling with a structural component 152, where the structural component includes one or more components for enabling coupling with the capsule portion 108 (see, e.g., shown in Figure 1 via fastening locations 112A and 112B in Figure 2). Figure 8 shows the first part of the sequence includes producing the loop 148 with mounting plate 150 being in the loop 148 (shown in Figure 7). The mounting plate 150 can include a PSA 156 that secures it within the loop, e.g., an adhesive can be placed on one or more surfaces of the mounting plate 150 that interface with the strain relief layer 126. The strain relief layer also includes a PSA 158 that secures the loop 148 in place.

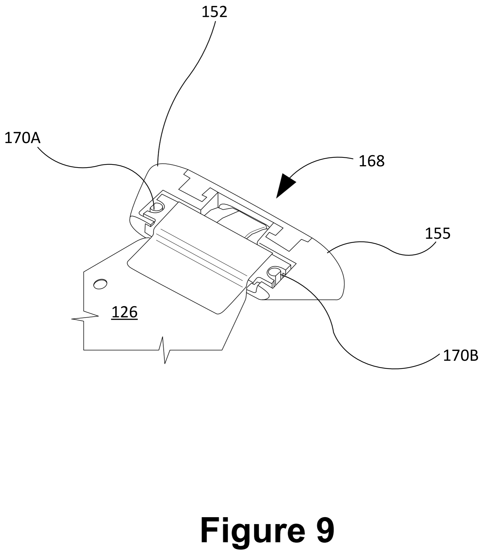

Figure 8 shows the second part of the sequence which shows the loop 148 wrapping around the mounting plate 150 and the loop 148 being secured to the portion of the strain relief layer 126 that has the second section width to produce a partially completed middle portion 168. In some embodiments, other ways of securing the loop are possible, including stitching, melting, clamping, etc. Figure 8 further illustrates the mounting plate 150 which includes semi-circular recesses 160A and 160B used for aligning and engaging the mounting plate 150 with the pins 170A and 170B of the structural component 152 (shown in Figure 9).

Figure 9 illustrates the third part of the sequence which shows the partially completed middle portion 168 being bonded with structural component 152. In some embodiments, the bonding occurs partially by the mounting plate 150 being laser welded with pins 170A and 170B of the structural component 152. In some embodiments, the bonding partially occurs when the partially completed middle portion 168 and the structural component 152 are overmolded by two separate shots of the LSR material, as shown in Figure 3.

Figure 9 further illustrates the semi-circular recess 160B engaging with pin 170A and semi-circular recess 160A engaging with pin 170B. The semi-circular recesses 160A and 160B are engaged with the pins 170A and 170B when a portion of each respective semi-circular recess is partially surrounding the respective pin. The engagement between the semi-circular recesses 160A and 160B with the pins 170A and 170B aligns the strain relief layer 126 in the appropriate position relative to the structural component 152 which in turn aligns the strain relief layer 126 with the capsule portion of the wrist-wearable device such that it strengthens the overall band portion 120 of the wrist-wearable device.

In some embodiments, the structural component 152 has a shape 153 that tapers to accommodate the larger mating junction 155 between the capsule portion 108 and the structural component 152 and the smaller end of the structural component 152 accommodates the width of the band portion 120. For example, the structural component tapers between junction 155 of the capsule portion 108 and the end of the structural component 152 that couples to the strain relief 126.

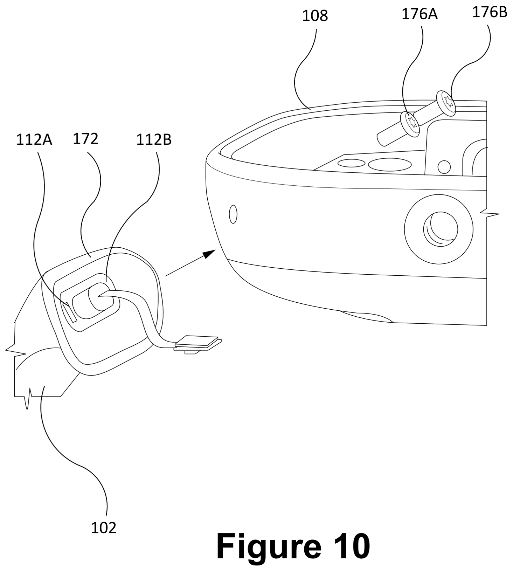

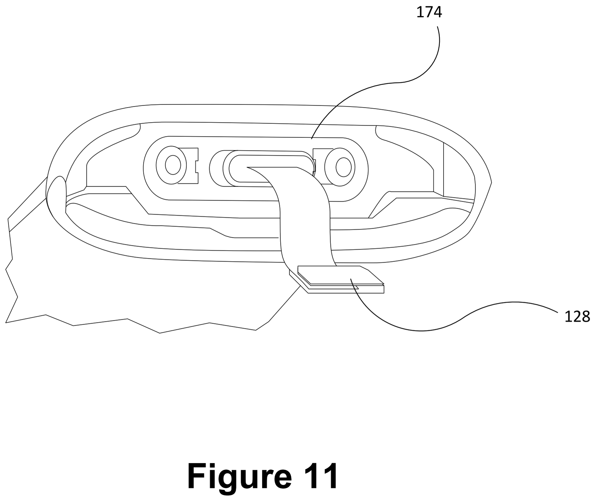

Figures 10-11 show the interfacing portion 172 of the band portion 120 of the wrist-wearable device 100, in accordance with some embodiments. Figure 10 also shows fasteners 176A and 176B that secure the band portion 120 to the capsule portion 108 via fastening locations 112A and 112B. The securing of the fasteners also applies the appropriate compression on the gasket 174 (shown in Figure 11) to produce the moisture and debris seal.

Figure 11 illustrates the interfacing portion 172 which shows a gasket 174 that seals the flexible printed circuit 128 from moisture and debris, when the band portion is coupled to the capsule portion 108. In some embodiments, the gasket (e.g., an LSR gasket) is injection molded with one of the two shots described in reference to Figure 3. In some embodiments, the gasket 174 is another suitable barrier, including a metal gasket, a composite gasket, a rubber gasket, or a glue gasket.

(A1) In accordance with some embodiments, a band portion of a wrist-wearable device comprises a flexible printed circuit (FPC) board (e.g., Figure 3 illustrates a flexible printed circuit 128). The band portion includes a mounting plate, a structural component coupled to a capsule portion of the wrist-wearable device, and one or more electrodes of electromyography sensors configured to couple to the flexible printed circuit board (e.g., Figure 2 illustrates a plurality of electrode receivers 114A-114F that are configured to receive a plurality of electrodes that are configured to record neuromuscular signals of a wearer). The band portion also includes a strain relief layer that is coupled to the flexible printed circuit board (e.g., Figure 3 shows a strain relief layer 126 comprised of a multifilament yarn spun from a liquid crystal polymer sheet). In some embodiments, a first portion of the strain relief layer wraps around at least the mounting plate such that the first portion of the strain relief layer is folded back onto and coupled to a second portion of the strain relief layer to secure the mounting plate within the strain relief layer and the mounting plate includes at least one recess for engaging the mounting plate with a structural component of the band portion, the mounting plate configured to couple the strain relief layer to the structural component. For example, Figures 6-7 show that the strain relief layer encapsulates a mounting plate 150 that interfaces structural component 152 that interfaces with the capsule portion 108.

(A2) In some embodiments of A1, the strain relief layer comprises a multifilament yarn spun from liquid crystal polymer (LCP).

(A3) In some embodiments of any one of A1-A2, the first portion of the strain relief layer is coupled to the second portion of the strain relief layer via an adhesive. For example,

Figure 7 shows the mounting plate 150 can include a PSA 156 that secures it within the loop, e.g., an adhesive can be placed on one or more surfaces of the mounting plate 150 that interface with the strain relief layer 126.

(A4) In some embodiments of A3, the adhesive is a heat-activated adhesive (HAF).

(A5) In some embodiments of any one of A1-A4, where the mounting plate is engaged with the structural component when the recess of the mounting plate is partially surrounding a pin of the structural component, where the pin in secured via laser welding. For example, Figure 9 shows that the mounting plate 150 is laser welded with pins 170A and 170B of the structural component 152.

(A6) In some embodiments of any one of A1-A5, a stiffening material is placed between the FPC board and the strain relief layer. For example, Figure 5 shows stiffening material 130A-130F and in some embodiments the PSA components 134A-134F, PSA components 136A-136F, and PSA components 138A-138F can also act as a further stiffening layer.

(A7) In some embodiments of A6, the stiffening material is coupled via an adhesive to the FPC and the strain relief layer. For example, Figure 5 shows PSA components 134A-134F, PSA components 136A-136F, and PSA components 138A-138F that indirectly or directly couple to the FPC 128.

(A8) In some embodiments of any one of A1-A7, the strain relief layer incudes one or more cutouts for placing the flexible printed circuit board. Figure 5 illustrates that the strain relief layer 126 includes one or more cutouts 140A-140B that guide one or more of the PSA components, stiffening components, and/or the flexible circuit.

(A9) In some embodiments of any one of A1-A8, the band portion is overmolded by liquid silicone rubber that encapsulates some of the band portion of the wrist-wearable device. For example, Figure 3 illustrates top shot 129A and bottom shot 129B that encapsulates some of the middle layer 124.

(A10) In some embodiments of any one of A1-A9, overmolding is a two-shot overmolding process. For example, Figure 2 illustrates top shot 129A and bottom shot 129B that encapsulates some of the middle layer 124.

(A11) In some embodiments of any one of A1-A10, the band portion includes a sealing gasket for inhibiting moisture and debris ingress at an electrical connection point between the band portion and a capsule portion of the wrist-wearable device. For example, Figures 10-11 illustrate an interfacing portion 172 showing a gasket 174 that seals the flexible printed circuit 128 from moisture and debris.

(B1) In accordance with some embodiments, a non-transitory, computer-readable storage medium including executable instructions that, when executed by one or more processors of a wrist-wearable device, cause the one or more processors to perform or cause performance of interacting with an extended-reality environment, where the-wrist-wearable device is configured in accordance with any one of A1-A11.

(C1) In accordance with some embodiments, a means for performing or causing performance of interacting with an extended-reality environment via a wrist-wearable device, where the-wrist-wearable device is configured in accordance with any one of A1-A11.

(D1) In accordance with some embodiments, a method for interacting with an extended-reality environment via a wrist-wearable device, where the wrist-wearable device is configured in accordance with any one of A1-A11.

(E1) In accordance with some embodiments, a wrist-wearable device comprises a band portion (e.g., a band portion 104 shown in Figure 1) including one or more electrical components (e.g., FPC 128 and a plurality of electrode receivers 114A-114F) configured to couple to a capsule portion with one or more additional electronic components. The wrist-wearable device includes a multifilament yarn spun from a liquid crystal polymer (LCP) layer coupled to the band portion and coupled to the one or more electrical components of the band portion (e.g., Figures 1-11 show a strain relief layer 126). The wrist-wearable device includes a mounting plate (e.g., mounting plate 150 shown in Figures 7-9) coupled to the first end of the multifilament yarn spun from the LCP layer. In some embodiments, the first end of the LCP layer is configured to wrap around the mounting plate back onto the LCP layer such that the first end of the LCP layer is configured to couple to a portion of the LCP layer via an adhesive. The wrist-wearable device includes a connector piece (e.g., structural component 152 shown in Figure 9) configured to couple to the mounting plate via pins (laser welded the pins, mounting plate and connector piece together).

(E2) In some embodiments of E1, where the adhesive is a heat-activated adhesive (HAF).

(E3) In some embodiments of any of E1-E2, where the strain relief layer incudes one or more cutouts for placing a flexible printed circuit board.

(E4) In some embodiments of any of E1-E3, where the band portion is overmolded by liquid silicone rubber that encapsulates some of the band portion of the wrist-wearable device.

(E5) In some embodiments of any of E1-E4, where overmolding is a two-shot overmolding process.

(E6) In some embodiments of any of E1-E4, where the band portion includes a sealing gasket for inhibiting moisture and debris ingress at an electrical connection point between the band portion and a capsule portion of the wrist-wearable device.

(F1) In accordance with some embodiments, a method includes coupling a strain relief layer to a mounting plate via wrapping a first portion of the strain relief layer around the mounting plate such that the first portion of the strain relief layer is folded back onto and coupled to a second portion of the strain relief layer and engaging the mounting plate with a structural component of a band portion of a wrist-wearable device via a recess of the mounting plate and a pin of the structural component.

(F2) In some embodiments of F1, the method further includes coupling the structural component of the band portion to a capsule portion of a wrist-wearable device.

(F3) In some embodiments of any of F1-F2, where engaging the mounting plate with the structural component of the band portion includes coupling the recess of the mounting plate such that the recess partially surrounds the pin of the structural component, and the method further includes coupling another recess of the mounting plate such that the other recess partially surrounds another pin of the structural component.

### Example Extended-Reality Systems

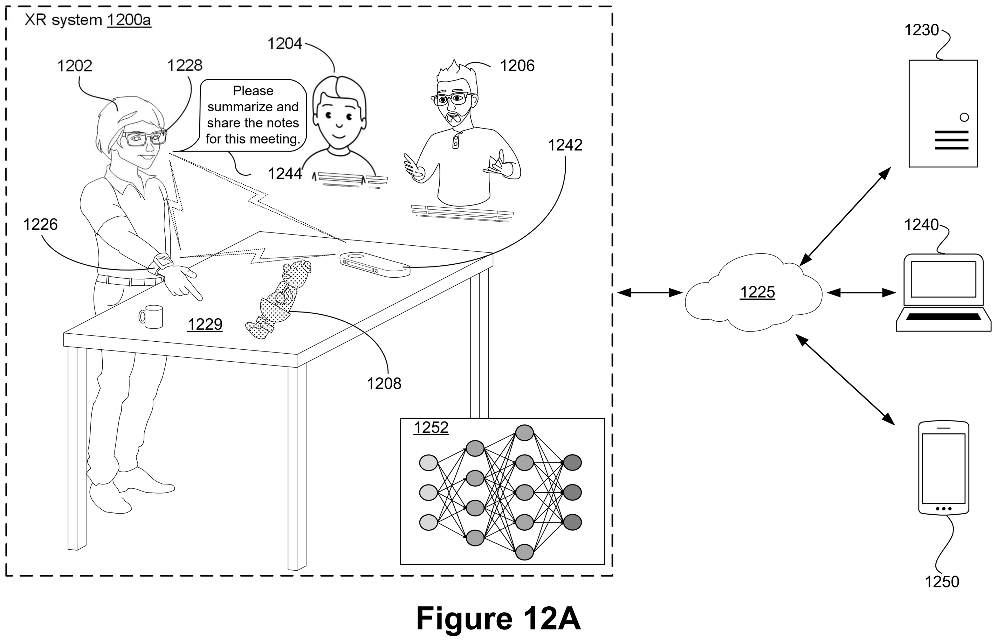

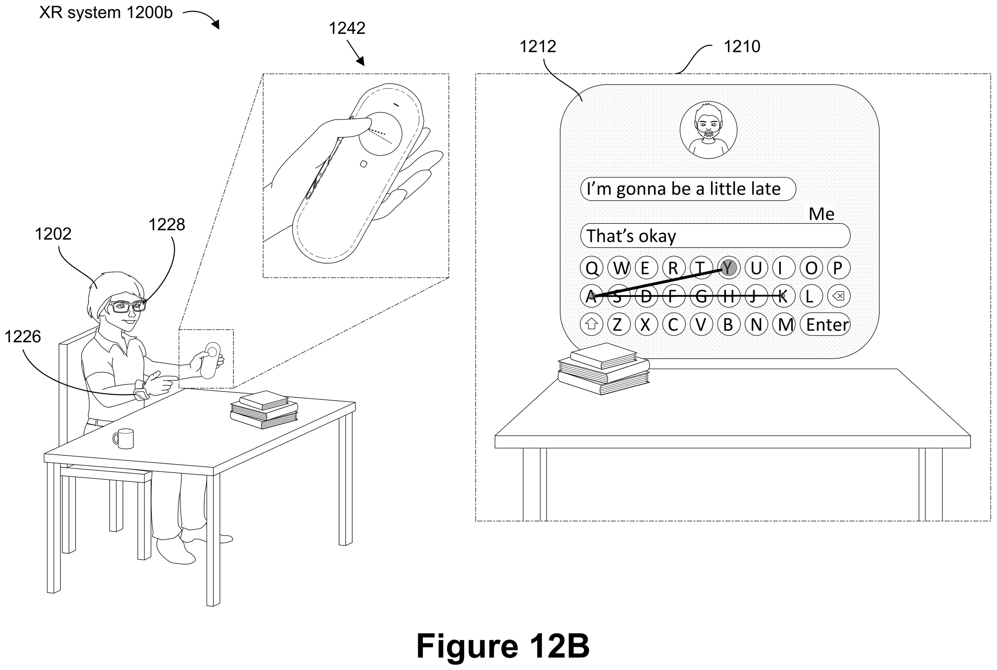

Figures 12A, 12B, 12C-1, and 12C-2, illustrate example XR systems that include AR and MR systems, in accordance with some embodiments. Figure 12A shows a first XR system 1200a and first example user interactions using a wrist-wearable device 1226, a head-wearable device (e.g., AR device 1228), and/or a HIPD 1242. Figure 12B shows a second XR system 1200b and second example user interactions using a wrist-wearable device 1226, AR device 1228, and/or an HIPD 1242. Figures 12C-1 and 12C-2 show a third MR system 1200c and third example user interactions using a wrist-wearable device 1226, a head-wearable device (e.g., an MR device such as a VR device), and/or an HIPD 1242. As the skilled artisan will appreciate upon reading the descriptions provided herein, the above-example AR and MR systems (described in detail below) can perform various functions and/or operations.

The wrist-wearable device 1226, the head-wearable devices, and/or the HIPD 1242 can communicatively couple via a network 1225 (e.g., cellular, near field, Wi-Fi, personal area network, wireless LAN). Additionally, the wrist-wearable device 1226, the head-wearable device, and/or the HIPD 1242 can also communicatively couple with one or more servers 1230, computers 1240 (e.g., laptops, computers), mobile devices 1250 (e.g., smartphones, tablets), and/or other electronic devices via the network 1225 (e.g., cellular, near field, Wi-Fi, personal area network, wireless LAN). Similarly, a smart textile-based garment, when used, can also communicatively couple with the wrist-wearable device 1226, the head-wearable device(s), the HIPD 1242, the one or more servers 1230, the computers 1240, the mobile devices 1250, and/or other electronic devices via the network 1225 to provide inputs.

Turning to Figure 12A, a user 1202 is shown wearing the wrist-wearable device 1226 and the AR device 1228 and having the HIPD 1242 on their desk. The wrist-wearable device 1226, the AR device 1228, and the HIPD 1242 facilitate user interaction with an AR environment. In particular, as shown by the first AR system 1200a, the wrist-wearable device 1226, the AR device 1228, and/or the HIPD 1242 cause presentation of one or more avatars 1204, digital representations of contacts 1206, and virtual objects 1208. As discussed below, the user 1202 can interact with the one or more avatars 1204, digital representations of the contacts 1206, and virtual objects 1208 via the wrist-wearable device 1226, the AR device 1228, and/or the HIPD 1242. In addition, the user 1202 is also able to directly view physical objects in the environment, such as a physical table 1229, through transparent lens(es) and waveguide(s) of the AR device 1228. Alternatively, an MR device could be used in place of the AR device 1228 and a similar user experience can take place, but the user would not be directly viewing physical objects in the environment, such as table 1229, and would instead be presented with a virtual reconstruction of the table 1229 produced from one or more sensors of the MR device (e.g., an outward facing camera capable of recording the surrounding environment).

The user 1202 can use any of the wrist-wearable device 1226, the AR device 1228 (e.g., through physical inputs at the AR device and/or built-in motion tracking of a user's extremities), a smart-textile garment, externally mounted extremity tracking device, the HIPD 1242 to provide user inputs, etc. For example, the user 1202 can perform one or more hand gestures that are detected by the wrist-wearable device 1226 (e.g., using one or more EMG sensors and/or IMUs built into the wrist-wearable device) and/or AR device 1228 (e.g., using one or more image sensors or cameras) to provide a user input. Alternatively, or additionally, the user 1202 can provide a user input via one or more touch surfaces of the wrist-wearable device 1226, the AR device 1228, and/or the HIPD 1242, and/or voice commands captured by a microphone of the wrist-wearable device 1226, the AR device 1228, and/or the HIPD 1242. The wrist-wearable device 1226, the AR device 1228, and/or the HIPD 1242 include an artificially intelligent digital assistant to help the user in providing a user input (e.g., completing a sequence of operations, suggesting different operations or commands, providing reminders, confirming a command). For example, the digital assistant can be invoked through an input occurring at the AR device 1228 (e.g., via an input at a temple arm of the AR device 1228). In some embodiments, the user 1202 can provide a user input via one or more facial gestures and/or facial expressions. For example, cameras of the wrist-wearable device 1226, the AR device 1228, and/or the HIPD 1242 can track the user 1202's eyes for navigating a user interface.

The wrist-wearable device 1226, the AR device 1228, and/or the HIPD 1242 can operate alone or in conjunction to allow the user 1202 to interact with the AR environment. In some embodiments, the HIPD 1242 is configured to operate as a central hub or control center for the wrist-wearable device 1226, the AR device 1228, and/or another communicatively coupled device. For example, the user 1202 can provide an input to interact with the AR environment at any of the wrist-wearable device 1226, the AR device 1228, and/or the HIPD 1242, and the HIPD 1242 can identify one or more back-end and front-end tasks to cause the performance of the requested interaction and distribute instructions to cause the performance of the one or more back-end and front-end tasks at the wrist-wearable device 1226, the AR device 1228, and/or the HIPD 1242. In some embodiments, a back-end task is a background-processing task that is not perceptible by the user (e.g., rendering content, decompression, compression, application-specific operations), and a front-end task is a user-facing task that is perceptible to the user (e.g., presenting information to the user, providing feedback to the user). The HIPD 1242 can perform the back-end tasks and provide the wrist-wearable device 1226 and/or the AR device 1228 operational data corresponding to the performed back-end tasks such that the wrist-wearable device 1226 and/or the AR device 1228 can perform the front-end tasks. In this way, the HIPD 1242, which has more computational resources and greater thermal headroom than the wrist-wearable device 1226 and/or the AR device 1228, performs computationally intensive tasks and reduces the computer resource utilization and/or power usage of the wrist-wearable device 1226 and/or the AR device 1228.

In the example shown by the first AR system 1200a, the HIPD 1242 identifies one or more back-end tasks and front-end tasks associated with a user request to initiate an AR video call with one or more other users (represented by the avatar 1204 and the digital representation of the contact 1206) and distributes instructions to cause the performance of the one or more back-end tasks and front-end tasks. In particular, the HIPD 1242 performs back-end tasks for processing and/or rendering image data (and other data) associated with the AR video call and provides operational data associated with the performed back-end tasks to the AR device 1228 such that the AR device 1228 performs front-end tasks for presenting the AR video call (e.g., presenting the avatar 1204 and the digital representation of the contact 1206).

The user 1202 can use any of the wrist-wearable device 1226, the AR device 1228 (e.g., through physical inputs at the AR device and/or built-in motion tracking of a user's extremities), a smart-textile garment, externally mounted extremity tracking device, the HIPD 1242 to provide user inputs, etc. For example, the user 1202 can perform one or more hand gestures that are detected by the wrist-wearable device 1226 (e.g., using one or more EMG sensors and/or IMUs built into the wrist-wearable device) and/or AR device 1228 (e.g., using one or more image sensors or cameras) to provide a user input. Alternatively, or additionally, the user 1202 can provide a user input via one or more touch surfaces of the wrist-wearable device 1226, the AR device 1228, and/or the HIPD 1242, and/or voice commands captured by a microphone of the wrist-wearable device 1226, the AR device 1228, and/or the HIPD 1242. The wrist-wearable device 1226, the AR device 1228, and/or the HIPD 1242 include an artificially intelligent digital assistant to help the user in providing a user input (e.g., completing a sequence of operations, suggesting different operations or commands, providing reminders, confirming a command). For example, the digital assistant can be invoked through an input occurring at the AR device 1228 (e.g., via an input at a temple arm of the AR device 1228). In some embodiments, the user 1202 can provide a user input via one or more facial gestures and/or facial expressions. For example, cameras of the wrist-wearable device 1226, the AR device 1228, and/or the HIPD 1242 can track the user 1202's eyes for navigating a user interface.

The wrist-wearable device 1226, the AR device 1228, and/or the HIPD 1242 can operate alone or in conjunction to allow the user 1202 to interact with the AR environment. In some embodiments, the HIPD 1242 is configured to operate as a central hub or control center for the wrist-wearable device 1226, the AR device 1228, and/or another communicatively coupled device. For example, the user 1202 can provide an input to interact with the AR environment at any of the wrist-wearable device 1226, the AR device 1228, and/or the HIPD 1242, and the HIPD 1242 can identify one or more back-end and front-end tasks to cause the performance of the requested interaction and distribute instructions to cause the performance of the one or more back-end and front-end tasks at the wrist-wearable device 1226, the AR device 1228, and/or the HIPD 1242. In some embodiments, a back-end task is a background-processing task that is not perceptible by the user (e.g., rendering content, decompression, compression, application-specific operations), and a front-end task is a user-facing task that is perceptible to the user (e.g., presenting information to the user, providing feedback to the user). The HIPD 1242 can perform the back-end tasks and provide the wrist-wearable device 1226 and/or the AR device 1228 operational data corresponding to the performed back-end tasks such that the wrist-wearable device 1226 and/or the AR device 1228 can perform the front-end tasks. In this way, the HIPD 1242, which has more computational resources and greater thermal headroom than the wrist-wearable device 1226 and/or the AR device 1228, performs computationally intensive tasks and reduces the computer resource utilization and/or power usage of the wrist-wearable device 1226 and/or the AR device 1228.

In the example shown by the first AR system 1200a, the HIPD 1242 identifies one or more back-end tasks and front-end tasks associated with a user request to initiate an AR video call with one or more other users (represented by the avatar 1204 and the digital representation of the contact 1206) and distributes instructions to cause the performance of the one or more back-end tasks and front-end tasks. In particular, the HIPD 1242 performs back-end tasks for processing and/or rendering image data (and other data) associated with the AR video call and provides operational data associated with the performed back-end tasks to the AR device 1228 such that the AR device 1228 performs front-end tasks for presenting the AR video call (e.g., presenting the avatar 1204 and the digital representation of the contact 1206).

In some embodiments, the HIPD 1242 can operate as a focal or anchor point for causing the presentation of information. This allows the user 1202 to be generally aware of where information is presented. For example, as shown in the first AR system 1200a, the avatar 1204 and the digital representation of the contact 1206 are presented above the HIPD 1242. In particular, the HIPD 1242 and the AR device 1228 operate in conjunction to determine a location for presenting the avatar 1204 and the digital representation of the contact 1206. In some embodiments, information can be presented within a predetermined distance from the HIPD 1242 (e.g., within five meters). For example, as shown in the first AR system 1200a, virtual object 1208 is presented on the desk some distance from the HIPD 1242. Similar to the above example, the HIPD 1242 and the AR device 1228 can operate in conjunction to determine a location for presenting the virtual object 1208. Alternatively, in some embodiments, presentation of information is not bound by the HIPD 1242. More specifically, the avatar 1204, the digital representation of the contact 1206, and the virtual object 1208 do not have to be presented within a predetermined distance of the HIPD 1242. While an AR device 1228 is described working with an HIPD, an MR headset can be interacted with in the same way as the AR device 1228.

User inputs provided at the wrist-wearable device 1226, the AR device 1228, and/or the HIPD 1242 are coordinated such that the user can use any device to initiate, continue, and/or complete an operation. For example, the user 1202 can provide a user input to the AR device 1228 to cause the AR device 1228 to present the virtual object 1208 and, while the virtual object 1208 is presented by the AR device 1228, the user 1202 can provide one or more hand gestures via the wrist-wearable device 1226 to interact and/or manipulate the virtual object 1208. While an AR device 1228 is described working with a wrist-wearable device 1226, an MR headset can be interacted with in the same way as the AR device 1228.

### Integration of Artificial Intelligence with XR Systems

Figure 12A illustrates an interaction in which an artificially intelligent virtual assistant can assist in requests made by a user 1202. The AI virtual assistant can be used to complete open-ended requests made through natural language inputs by a user 1202. For example, in Figure 12A the user 1202 makes an audible request 1244 to summarize the conversation and then share the summarized conversation with others in the meeting. In addition, the AI virtual assistant is configured to use sensors of the XR system (e.g., cameras of an XR headset, microphones, and various other sensors of any of the devices in the system) to provide contextual prompts to the user for initiating tasks.

Figure 12A also illustrates an example neural network 1252 used in Artificial Intelligence applications. Uses of Artificial Intelligence (AI) are varied and encompass many different aspects of the devices and systems described herein. AI capabilities cover a diverse range of applications and deepen interactions between the user 1202 and user devices (e.g., the AR device 1228, an MR device 1232, the HIPD 1242, the wrist-wearable device 1226). The AI discussed herein can be derived using many different training techniques. While the primary AI model example discussed herein is a neural network, other AI models can be used. Non-limiting examples of AI models include artificial neural networks (ANNs), deep neural networks (DNNs), convolution neural networks (CNNs), recurrent neural networks (RNNs), large language models (LLMs), long short-term memory networks, transformer models, decision trees, random forests, support vector machines, k-nearest neighbors, genetic algorithms, Markov models, Bayesian networks, fuzzy logic systems, and deep reinforcement learnings, etc. The AI models can be implemented at one or more of the user devices, and/or any other devices described herein. For devices and systems herein that employ multiple AI models, different models can be used depending on the task. For example, for a natural-language artificially intelligent virtual assistant, an LLM can be used and for the object detection of a physical environment, a DNN can be used instead.

In another example, an AI virtual assistant can include many different AI models and based on the user's request, multiple AI models may be employed (concurrently, sequentially or a combination thereof). For example, an LLM-based AI model can provide instructions for helping a user follow a recipe and the instructions can be based in part on another AI model that is derived from an ANN, a DNN, an RNN, etc. that is capable of discerning what part of the recipe the user is on (e.g., object and scene detection).

As AI training models evolve, the operations and experiences described herein could potentially be performed with different models other than those listed above, and a person skilled in the art would understand that the list above is non-limiting.

A user 1202 can interact with an AI model through natural language inputs captured by a voice sensor, text inputs, or any other input modality that accepts natural language and/or a corresponding voice sensor module. In another instance, input is provided by tracking the eye gaze of a user 1202 via a gaze tracker module. Additionally, the AI model can also receive inputs beyond those supplied by a user 1202. For example, the AI can generate its response further based on environmental inputs (e.g., temperature data, image data, video data, ambient light data, audio data, GPS location data, inertial measurement (i.e., user motion) data, pattern recognition data, magnetometer data, depth data, pressure data, force data, neuromuscular data, heart rate data, temperature data, sleep data) captured in response to a user request by various types of sensors and/or their corresponding sensor modules. The sensors' data can be retrieved entirely from a single device (e.g., AR device 1228) or from multiple devices that are in communication with each other (e.g., a system that includes at least two of an AR device 1228, an MR device 1232, the HIPD 1242, the wrist-wearable device 1226, etc.). The AI model can also access additional information (e.g., one or more servers 1230, the computers 1240, the mobile devices 1250, and/or other electronic devices) via a network 1225.

A non-limiting list of Al-enhanced functions includes but is not limited to image recognition, speech recognition (e.g., automatic speech recognition), text recognition (e.g., scene text recognition), pattern recognition, natural language processing and understanding, classification, regression, clustering, anomaly detection, sequence generation, content generation, and optimization. In some embodiments, Al-enhanced functions are fully or partially executed on cloud-computing platforms communicatively coupled to the user devices (e.g., the AR device 1228, an MR device 1232, the HIPD 1242, the wrist-wearable device 1226) via the one or more networks. The cloud-computing platforms provide scalable computing resources, distributed computing, managed AI services, interference acceleration, pre-trained models, APIs and/or other resources to support comprehensive computations required by the AI-enhanced function.

Example outputs stemming from the use of an AI model can include natural language responses, mathematical calculations, charts displaying information, audio, images, videos, texts, summaries of meetings, predictive operations based on environmental factors, classifications, pattern recognitions, recommendations, assessments, or other operations. In some embodiments, the generated outputs are stored on local memories of the user devices (e.g., the AR device 1228, an MR device 1232, the HIPD 1242, the wrist-wearable device 1226), storage options of the external devices (servers, computers, mobile devices, etc.), and/or storage options of the cloud-computing platforms.

The AI-based outputs can be presented across different modalities (e.g., audio-based, visual-based, haptic-based, and any combination thereof) and across different devices of the XR system described herein. Some visual-based outputs can include the displaying of information on XR augments of an XR headset, user interfaces displayed at a wrist-wearable device, laptop device, mobile device, etc. On devices with or without displays (e.g., HIPD 1242), haptic feedback can provide information to the user 1202. An AI model can also use the inputs described above to determine the appropriate modality and device(s) to present content to the user (e.g., a user walking on a busy road can be presented with an audio output instead of a visual output to avoid distracting the user 1202).

### Example Augmented Reality Interaction

Figure 12B shows the user 1202 wearing the wrist-wearable device 1226 and the AR device 1228 and holding the HIPD 1242. In the second AR system 1200b, the wrist-wearable device 1226, the AR device 1228, and/or the HIPD 1242 are used to receive and/or provide one or more messages to a contact of the user 1202. In particular, the wrist-wearable device 1226, the AR device 1228, and/or the HIPD 1242 detect and coordinate one or more user inputs to initiate a messaging application and prepare a response to a received message via the messaging application.

In some embodiments, the user 1202 initiates, via a user input, an application on the wrist-wearable device 1226, the AR device 1228, and/or the HIPD 1242 that causes the application to initiate on at least one device. For example, in the second AR system 1200b the user 1202 performs a hand gesture associated with a command for initiating a messaging application (represented by messaging user interface 1212); the wrist-wearable device 1226 detects the hand gesture; and, based on a determination that the user 1202 is wearing the AR device 1228, causes the AR device 1228 to present a messaging user interface 1212 of the messaging application. The AR device 1228 can present the messaging user interface 1212 to the user 1202 via its display (e.g., as shown by user 1202's field of view 1210). In some embodiments, the application is initiated and can be run on the device (e.g., the wrist-wearable device 1226, the AR device 1228, and/or the HIPD 1242) that detects the user input to initiate the application, and the device provides another device operational data to cause the presentation of the messaging application. For example, the wrist-wearable device 1226 can detect the user input to initiate a messaging application, initiate and run the messaging application, and provide operational data to the AR device 1228 and/or the HIPD 1242 to cause presentation of the messaging application. Alternatively, the application can be initiated and run at a device other than the device that detected the user input. For example, the wrist-wearable device 1226 can detect the hand gesture associated with initiating the messaging application and cause the HIPD 1242 to run the messaging application and coordinate the presentation of the messaging application.

Further, the user 1202 can provide a user input provided at the wrist-wearable device 1226, the AR device 1228, and/or the HIPD 1242 to continue and/or complete an operation initiated at another device. For example, after initiating the messaging application via the wrist-wearable device 1226 and while the AR device 1228 presents the messaging user interface 1212, the user 1202 can provide an input at the HIPD 1242 to prepare a response (e.g., shown by the swipe gesture performed on the HIPD 1242). The user 1202's gestures performed on the HIPD 1242 can be provided and/or displayed on another device. For example, the user 1202's swipe gestures performed on the HIPD 1242 are displayed on a virtual keyboard of the messaging user interface 1212 displayed by the AR device 1228.

In some embodiments, the wrist-wearable device 1226, the AR device 1228, the HIPD 1242, and/or other communicatively coupled devices can present one or more notifications to the user 1202. The notification can be an indication of a new message, an incoming call, an application update, a status update, etc. The user 1202 can select the notification via the wrist-wearable device 1226, the AR device 1228, or the HIPD 1242 and cause presentation of an application or operation associated with the notification on at least one device. For example, the user 1202 can receive a notification that a message was received at the wrist-wearable device 1226, the AR device 1228, the HIPD 1242, and/or other communicatively coupled device and provide a user input at the wrist-wearable device 1226, the AR device 1228, and/or the HIPD 1242 to review the notification, and the device detecting the user input can cause an application associated with the notification to be initiated and/or presented at the wrist-wearable device 1226, the AR device 1228, and/or the HIPD 1242.

While the above example describes coordinated inputs used to interact with a messaging application, the skilled artisan will appreciate upon reading the descriptions that user inputs can be coordinated to interact with any number of applications including, but not limited to, gaming applications, social media applications, camera applications, web-based applications, financial applications, etc. For example, the AR device 1228 can present to the user 1202 game application data and the HIPD 1242 can use a controller to provide inputs to the game. Similarly, the user 1202 can use the wrist-wearable device 1226 to initiate a camera of the AR device 1228, and the user can use the wrist-wearable device 1226, the AR device 1228, and/or the HIPD 1242 to manipulate the image capture (e.g., zoom in or out, apply filters) and capture image data.

While an AR device 1228 is shown being capable of certain functions, it is understood that an AR device can be an AR device with varying functionalities based on costs and market demands. For example, an AR device may include a single output modality such as an audio output modality. In another example, the AR device may include a low-fidelity display as one of the output modalities, where simple information (e.g., text and/or low-fidelity images/video) is capable of being presented to the user. In yet another example, the AR device can be configured with face-facing light emitting diodes (LEDs) configured to provide a user with information, e.g., an LED around the right-side lens can illuminate to notify the wearer to turn right while directions are being provided or an LED on the left-side can illuminate to notify the wearer to turn left while directions are being provided. In another embodiment, the AR device can include an outward-facing projector such that information (e.g., text information, media) may be displayed on the palm of a user's hand or other suitable surface (e.g., a table, whiteboard). In yet another embodiment, information may also be provided by locally dimming portions of a lens to emphasize portions of the environment in which the user's attention should be directed. Some AR devices can present AR augments either monocularly or binocularly (e.g., an AR augment can be presented at only a single display associated with a single lens as opposed presenting an AR augmented at both lenses to produce a binocular image). In some instances an AR device capable of presenting AR augments binocularly can optionally display AR augments monocularly as well (e.g., for power-saving purposes or other presentation considerations). These examples are non-exhaustive and features of one AR device described above can be combined with features of another AR device described above. While features and experiences of an AR device have been described generally in the preceding sections, it is understood that the described functionalities and experiences can be applied in a similar manner to an MR headset, which is described below in the proceeding sections.

### Example Mixed Reality Interaction

Turning to Figures 12C-1 and 12C-2, the user 1202 is shown wearing the wrist-wearable device 1226 and an MR device 1232 (e.g., a device capable of providing either an entirely VR experience or an MR experience that displays object(s) from a physical environment at a display of the device) and holding the HIPD 1242. In the third MR system 1200c, the wrist-wearable device 1226, the MR device 1232, and/or the HIPD 1242 are used to interact within an MR environment, such as a VR game or other MR/VR application. While the MR device 1232 presents a representation of a VR game (e.g., first MR game environment 1220) to the user 1202, the wrist-wearable device 1226, the MR device 1232, and/or the HIPD 1242 detect and coordinate one or more user inputs to allow the user 1202 to interact with the VR game.

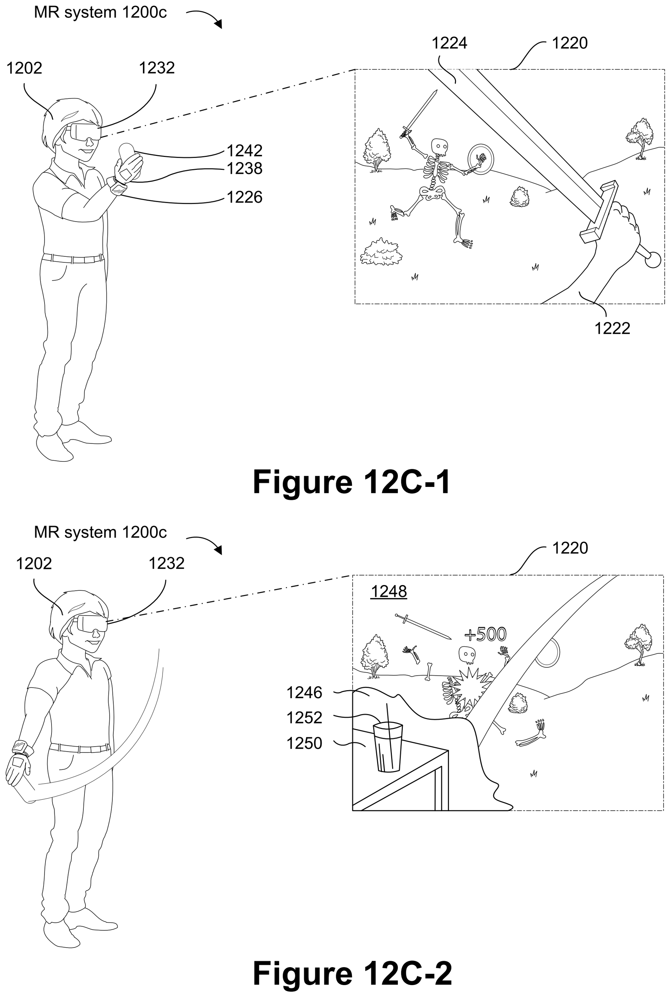

In some embodiments, the user 1202 can provide a user input via the wrist-wearable device 1226, the MR device 1232, and/or the HIPD 1242 that causes an action in a corresponding MR environment. For example, the user 1202 in the third MR system 1200c (shown in Figure 12C-1) raises the HIPD 1242 to prepare for a swing in the first MR game environment 1220. The MR device 1232, responsive to the user 1202 raising the HIPD 1242, causes the MR representation of the user 1222 to perform a similar action (e.g., raise a virtual object, such as a virtual sword 1224). In some embodiments, each device uses respective sensor data and/or image data to detect the user input and provide an accurate representation of the user 1202's motion. For example, image sensors (e.g., SLAM cameras or other cameras) of the HIPD 1242 can be used to detect a position of the HIPD 1242 relative to the user 1202's body such that the virtual object can be positioned appropriately within the first MR game environment 1220; sensor data from the wrist-wearable device 1226 can be used to detect a velocity at which the user 1202 raises the HIPD 1242 such that the MR representation of the user 1222 and the virtual sword 1224 are synchronized with the user 1202's movements; and image sensors of the MR device 1232 can be used to represent the user 1202's body, boundary conditions, or real-world objects within the first MR game environment 1220.

In Figure 12C-2, the user 1202 performs a downward swing while holding the HIPD 1242. The user 1202's downward swing is detected by the wrist-wearable device 1226, the MR device 1232, and/or the HIPD 1242 and a corresponding action is performed in the first MR game environment 1220. In some embodiments, the data captured by each device is used to improve the user's experience within the MR environment. For example, sensor data of the wrist-wearable device 1226 can be used to determine a speed and/or force at which the downward swing is performed and image sensors of the HIPD 1242 and/or the MR device 1232 can be used to determine a location of the swing and how it should be represented in the first MR game environment 1220, which, in turn, can be used as inputs for the MR environment (e.g., game mechanics, which can use detected speed, force, locations, and/or aspects of the user 1202's actions to classify a user's inputs (e.g., user performs a light strike, hard strike, critical strike, glancing strike, miss) or calculate an output (e.g., amount of damage)).

Figure 12C-2 further illustrates that a portion of the physical environment is reconstructed and displayed at a display of the MR device 1232 while the MR game environment 1220 is being displayed. In this instance, a reconstruction of the physical environment 1246 is displayed in place of a portion of the MR game environment 1220 when object(s) in the physical environment are potentially in the path of the user (e.g., a collision with the user and an object in the physical environment are likely). Thus, this example MR game environment 1220 includes (i) an immersive VR portion 1248 (e.g., an environment that does not have a corollary counterpart in a nearby physical environment) and (ii) a reconstruction of the physical environment 1246 (e.g., table 1250 and cup). While the example shown here is an MR environment that shows a reconstruction of the physical environment to avoid collisions, other uses of reconstructions of the physical environment can be used, such as defining features of the virtual environment based on the surrounding physical environment (e.g., a virtual column can be placed based on an object in the surrounding physical environment (e.g., a tree)).

While the wrist-wearable device 1226, the MR device 1232, and/or the HIPD 1242 are described as detecting user inputs, in some embodiments, user inputs are detected at a single device (with the single device being responsible for distributing signals to the other devices for performing the user input). For example, the HIPD 1242 can operate an application for generating the first MR game environment 1220 and provide the MR device 1232 with corresponding data for causing the presentation of the first MR game environment 1220, as well as detect the user 1202's movements (while holding the HIPD 1242) to cause the performance of corresponding actions within the first MR game environment 1220. Additionally or alternatively, in some embodiments, operational data (e.g., sensor data, image data, application data, device data, and/or other data) of one or more devices is provided to a single device (e.g., the HIPD 1242) to process the operational data and cause respective devices to perform an action associated with processed operational data.

In some embodiments, the user 1202 can wear a wrist-wearable device 1226, wear an MR device 1232, wear smart textile-based garments 1238 (e.g., wearable haptic gloves), and/or hold an HIPD 1242 device. In this embodiment, the wrist-wearable device 1226, the MR device 1232, and/or the smart textile-based garments 1238 are used to interact within an MR environment (e.g., any AR or MR system described above in reference to Figures 12A-12B). While the MR device 1232 presents a representation of an MR game (e.g., second MR game environment 1220) to the user 1202, the wrist-wearable device 1226, the MR device 1232, and/or the smart textile-based garments 1238 detect and coordinate one or more user inputs to allow the user 1202 to interact with the MR environment.

In some embodiments, the user 1202 can provide a user input via the wrist-wearable device 1226, an HIPD 1242, the MR device 1232, and/or the smart textile-based garments 1238 that causes an action in a corresponding MR environment. In some embodiments, each device uses respective sensor data and/or image data to detect the user input and provide an accurate representation of the user 1202's motion. While four different input devices are shown (e.g., a wrist-wearable device 1226, an MR device 1232, an HIPD 1242, and a smart textile-based garment 1238) each one of these input devices entirely on its own can provide inputs for fully interacting with the MR environment. For example, the wrist-wearable device can provide sufficient inputs on its own for interacting with the MR environment. In some embodiments, if multiple input devices are used (e.g., a wrist-wearable device and the smart textile-based garment 1238) sensor fusion can be utilized to ensure inputs are correct. While multiple input devices are described, it is understood that other input devices can be used in conjunction or on their own instead, such as but not limited to external motion-tracking cameras, other wearable devices fitted to different parts of a user, apparatuses that allow for a user to experience walking in an MR environment while remaining substantially stationary in the physical environment, etc.

As described above, the data captured by each device is used to improve the user's experience within the MR environment. Although not shown, the smart textile-based garments 1238 can be used in conjunction with an MR device and/or an HIPD 1242.

While some experiences are described as occurring on an AR device and other experiences are described as occurring on an MR device, one skilled in the art would appreciate that experiences can be ported over from an MR device to an AR device, and vice versa.

Some definitions of devices and components that can be included in some or all of the example devices discussed are defined here for ease of reference. A skilled artisan will appreciate that certain types of the components described may be more suitable for a particular set of devices, and less suitable for a different set of devices. But subsequent reference to the components defined here should be considered to be encompassed by the definitions provided.

In some embodiments example devices and systems, including electronic devices and systems, will be discussed. Such example devices and systems are not intended to be limiting, and one of skill in the art will understand that alternative devices and systems to the example devices and systems described herein may be used to perform the operations and construct the systems and devices that are described herein.

As described herein, an electronic device is a device that uses electrical energy to perform a specific function. It can be any physical object that contains electronic components such as transistors, resistors, capacitors, diodes, and integrated circuits. Examples of electronic devices include smartphones, laptops, digital cameras, televisions, gaming consoles, and music players, as well as the example electronic devices discussed herein. As described herein, an intermediary electronic device is a device that sits between two other electronic devices, and/or a subset of components of one or more electronic devices and facilitates communication, and/or data processing and/or data transfer between the respective electronic devices and/or electronic components.

The foregoing descriptions of Figures 12A-12C-2 provided above are intended to augment the description provided in reference to Figures 1-11. While terms in the following description may not be identical to terms used in the foregoing description, a person having ordinary skill in the art would understand these terms to have the same meaning.

Any data collection performed by the devices described herein and/or any devices configured to perform or cause the performance of the different embodiments described above in reference to any of the Figures, hereinafter the "devices," is done with user consent and in a manner that is consistent with all applicable privacy laws. Users are given options to allow the devices to collect data, as well as the option to limit or deny collection of data by the devices. A user is able to opt in or opt out of any data collection at any time. Further, users are given the option to request the removal of any collected data.

It will be understood that, although the terms "first," "second," etc. may be used herein to describe various elements, these elements should not be limited by these terms. These terms are only used to distinguish one element from another.

The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" can be construed to mean "when" or "upon" or "in response to determining" or "in accordance with a determination" or "in response to detecting," that a stated condition precedent is true, depending on the context. Similarly, the phrase "if it is determined [that a stated condition precedent is true]" or "if [a stated condition precedent is true]" or "when [a stated condition precedent is true]" can be construed to mean "upon determining" or "in response to determining" or "in accordance with a determination" or "upon detecting" or "in response to detecting" that the stated condition precedent is true, depending on the context.

The foregoing description, for purpose of explanation, has been described with reference to specific embodiments. However, the illustrative discussions above are not intended to be exhaustive or to limit the claims to the precise forms disclosed. Many modifications and variations are possible in view of the above teachings. The embodiments were chosen and described in order to best explain principles of operation and practical applications, to thereby enable others skilled in the art.

## Claims

1. A band portion of a wrist-wearable device, comprising:
a flexible printed circuit, FPC, board;
a mounting plate;
a structural component coupled to a capsule portion of the wrist-wearable device;
one or more electromyography sensors, wherein the one or more electromyography sensors each include one or more electrodes configured to couple to the FPC board; and
a strain relief layer coupled to the FPC board, wherein:
a first portion of the strain relief layer wraps around at least the mounting plate such that the first portion of the strain relief layer is folded back onto and coupled to a second portion of the strain relief layer to secure the mounting plate within the strain relief layer; and
the mounting plate includes at least one recess for engaging the mounting plate with a structural component of the band portion, the mounting plate configured to couple the strain relief layer to the structural component.

2. The band portion of the wrist-wearable device of claim 1, wherein the strain relief layer comprises a multifilament yarn spun from liquid crystal polymer, LCP.

3. The band portion of the wrist-wearable device of claim 1 or claim 2, wherein the first portion of the strain relief layer is coupled to the second portion of the strain relief layer via an adhesive,
and optionally wherein the adhesive is a heat-activated adhesive, HAF.

4. The band portion of the wrist-wearable device of any preceding claim, wherein the mounting plate is engaged with the structural component when the recess of the mounting plate is partially surrounding a pin of the structural component, where the pin secured via laser welding.

5. The band portion of the wrist-wearable device of any preceding claim, wherein a stiffening material is placed between the FPC board and the strain relief layer, and optionally wherein the stiffening material is coupled via an adhesive to the FPC and the strain relief layer.

6. The band portion of the wrist-wearable device of any preceding claim, wherein the strain relief layer incudes one or more cutouts for placing the FPC board.

7. The band portion of the wrist-wearable device of any preceding claim, wherein the band portion is overmolded by liquid silicone rubber that encapsulates some of the band portion of the wrist-wearable device.

8. The band portion of the wrist-wearable device of any preceding claim, wherein overmolding is a two shot overmolding process.

9. The band portion of the wrist-wearable device of any preceding claim, wherein the band portion includes a sealing gasket for inhibiting moisture and debris ingress at an electrical connection point between the band portion and a capsule portion of the wrist-wearable device, and/or
wherein the strain relief layer incudes one or more cutouts for placing a flexible printed circuit board.

10. A wrist-wearable device comprising:
a band portion including one or more electrical components, wherein the band portion is configured to couple to a capsule portion with one or more additional electronic components;
a multifilament yarn spun from liquid crystal polymer, LCP, layer coupled to the band portion and coupled to the one or more electrical components of the band portion;
a mounting plate coupled to a first end of the multifilament yarn spun from LCP layer, wherein:
a first end of the LCP layer is configured to wrap around the mounting plate back onto the LCP layer such that the first end of the LCP layer is configured to couple to a portion of the LCP layer via an adhesive; and
a connector piece configured to couple to the mounting plate via pins.

11. The wrist-wearable device of claim 10, wherein the adhesive is a heat-activated adhesive, HAF.

12. The wrist-wearable device of claim 10 or claim 11, wherein the band portion is overmolded by liquid silicone rubber that encapsulates some of the band portion of the wrist-wearable device.

13. The band portion of the wrist-wearable device of any of claims 10 to 12, wherein overmolding is a two shot overmolding process,
and/or
wherein the band portion includes a sealing gasket for inhibiting moisture and debris ingress at an electrical connection point between the band portion and a capsule portion of the wrist-wearable device.

14. A method, comprising:
coupling a strain relief layer to a mounting plate via wrapping a first portion of the strain relief layer around the mounting plate such that the first portion of the strain relief layer is folded back onto and coupled to a second portion of the strain relief layer; and
engaging the mounting plate with a structural component of a band portion of a wrist-wearable device via a recess of the mounting plate and a pin of the structural component.

15. The method of claim 14, further comprising coupling the structural component of the band portion to a capsule portion of a wrist-wearable device,
and/or
wherein engaging the mounting plate with the structural component of the band portion includes coupling the recess of the mounting plate such that the recess partially surrounds the pin of the structural component and the method further includes coupling another recess of the mounting plate such that the other recess partially surrounds another pin of the structural component.
